(19) Europäisches Patentamt
European Patent Office
Office européen des brevets

(11) EP 2 976 061 B1

# (12) EUROPEAN PATENT SPECIFICATION

(45) Date of publication and mention of the grant of the patent:
**16.10.2019 Bulletin 2019/42**

(21) Application number: **14769500.1**

(22) Date of filing: **16.03.2014**

(51) Int Cl.:
***A61K 8/97*** *(2017.01)*
***A61P 1/02*** *(2006.01)*
***A61K 8/34*** *(2006.01)*
***A61K 8/44*** *(2006.01)*
***A61Q 11/00*** *(2006.01)*

(86) International application number:
**PCT/IL2014/050290**

(87) International publication number:
**WO 2014/147613 (25.09.2014 Gazette 2014/39)**

## (54) COMBINATIONS OF N-ACETYL CYSTEINE DERIVATIVES AND CRANBERRY POLYPHENOLS IN COMPOSITIONS AND METHODS FOR PREVENTING AND TREATING PERIODONTAL DISEASES AND PERI-IMPLATITIS

KOMBINATIONEN AUS N-ACETYLCYSTEINDERIVATEN UND MOOSBEERPOLYPHENOLEN IN ZUSAMMENSETZUNGEN UND VERFAHREN ZUR PRÄVENTION UND BEHANDLUNG PERIODONTALEN KRANKHEITEN UND PERIIMPLATITIS

COMBINAISON DE DÉRIVÉS DE N-ACÉTYLCYSTÉINE ET DE POLYPHÉNOLS DE CANNEBERGE DANS DES COMPOSITIONS ET DES PROCÉDÉS POUR PRÉVENIR ET TRAITER DES MALADIES PARODONTIQUES ET UNE PÉRI-IMPLANTITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2013 US 201361802763 P**

(43) Date of publication of application:
**27.01.2016 Bulletin 2016/04**

(73) Proprietor: **Dentalmed Pharm Holding Ltd.**
**1650 Beersel (BE)**

(72) Inventors:
- **DANENBERG, Noam**
  **4520404 Hod Hasharon (IL)**
- **HALFON, Philippe**
  **F-13008 Marseille (FR)**

(74) Representative: **Dehns**
**St. Bride's House**
**10 Salisbury Square**
**London EC4Y 8JD (GB)**

(56) References cited:
**EP-A1- 0 465 921**
**WO-A1-2006/108430**
**US-A1- 2003 170 319**
**US-A1- 2006 134 014**
**US-A1- 2007 178 216**

- **Laetitia Bonifait ET AL: "Cranberry polyphenols: potential benefits for dental caries and periodontal disease", Journal (Canadian Dental Association), 1 January 2010 (2010-01-01), page a130, XP055284363, Canada Retrieved from the Internet: URL:http://www.jcda.ca/sites/default/files /a130/a130.pdf**
- **ZHAO TIEMEI ET AL: "N-acetylcysteine inhibit biofilms produced by Pseudomonas aeruginosa", BMC MICROBIOLOGY, BIOMED CENTRAL LTD, GB, vol. 10, no. 1, 12 May 2010 (2010-05-12), page 140, XP021073024, ISSN: 1471-2180, DOI: 10.1186/1471-2180-10-140**
- **DATABASE GNPD [Online] MINTEL; January 2011 (2011-01), Mintel: "Energy Activator with Superfoods", XP002759323, Database accession no. 1477929**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to combined therapy of N-acetyl cysteine and polyphenols, specifically cranberry polyphenols. More specifically, the invention provides compositions and methods for preventing and treating a periodontal disease, specifically gingivitis, peri-mucositis, periodontitis and peri-implantitis.

### Background of the Invention

The supporting tissue around the teeth and dental implants are affected by a spectrum of periodontal diseases, ranging from non-destructive forms like gingivitis and peri-implant mucositis, which are essentially reversible conditions, to destructive forms like periodontitis and peri-implantitis, which are typically chronic, progressive and essentially irreversible damage conditions.

Due to the bacterial infection, the periodontal tissues become inflamed and are slowly destroyed by the action of the inflammatory process. If left untreated the teeth lose their ligamentous support to the alveolar bone and become mobile and eventually lost.

Gingivitis is a non-destructive periodontal disease, in which the gum tissue develops inflammation. The most common form of gingivitis, and the most common form of periodontal disease in general, happens as a response to bacterial biofilms, also referred to as plaque, adherent to tooth surfaces, termed plaque-induced gingivitis. In the absence of treatment, gingivitis may progress to periodontitis, which is a destructive form of periodontal disease.

Periodontitis is a chronic infectious disease of the supporting tissues of the teeth. The disease is common worldwide and is the main cause for tooth loss in adults. The estimated prevalence of severe periodontitis is about 20% of the world adult population.

Peri-implantitis is a clinical manifestation where clinically and radiologically evident loss of the bony support around the implant occurs, combined with an inflammatory reaction of the peri-implant mucosa.

Bacteria of various and numerous species are common inhabitants of the mouth normal flora. Teeth provide hard non-shedding surfaces for the development of bacterial specific niches that are the primary cause of periodontal and other oral diseases. In general, bacteria living on hard surfaces in fluid environment such as teeth tend to aggregate in films bound together in a polysaccharide matrix with other organic and inorganic materials. These complexes are termed oral biofilms (e.g. biofilms) or dental plaque.

Biofilm formation around the periodontal tissue is a spatiotemporal process involving staged colonization of various bacterial species. Early colonizers are able to adhere directly to teeth surfaces and late colonizers bind to the former bacterial layer. Over 1500 bacterial species have been identified as part of the periodontal tissue biofilms. Of them, only few specific species are associated with periodontal disease, such as *Porphyromonas gingivalis, Tannerella forsythia, Aggregibcater actynomycetemcomitans* and others.

Inflammatory and immune processes operate in the periodontal tissue to protect against microbial attack and prevent microorganisms or their damaging products from spreading into or invading the tissues. These processes involve components including gingival crevicular fluid (GCF), complement system, antibodies, polymorphonuclear neutrophils (PMNs), cytokines and macrophages and lymphocytes (B and T). These host defense reactions are however also considered harmful to the host. The inflammation process can damage connective tissue structures eventually causing loss of periodontal tissue and alveolar bone destruction.

The main goal of the treatment of periodontal diseases is to decrease the bacterial load around the periodontal tissues. Since the bacteria form a biofilm, the mainstay of the treatments in cases of periodontal diseases includes meticulous oral hygiene and mechanical debridement. The components of mechanical debridement are scaling, root planning and debridement of the soft tissues. This procedure is usually followed by clinical reevaluation and sites that are still affected are treated with periodontal surgery. This process is invasive and time consuming, requiring repeated treatment sessions.

Therefore, different methods were sought to simplify and minimize the periodontal treatment by adding different chemical agents to the standard of care regimen. These measures includes the use of antimicrobial agents such as chlorhexidine in a gel formulation (Chlosite) or in a slow release device protein (Periochip), systemic antibiotics such as tetracycline, amoxicillin, amoxicillin+metronidazole, doxycillin and minocycline, as well as locally applied antibiotics, e.g. tetracycline fibers, metronidazole gel and minocycline microcapsules.

It is noted that the current available materials are of limited value. The use of chlorhexidine is limited by local side effects and poor penetration due to its cationic nature. The use of local antibiotics may result in emergence of resistant bacteria.

N-acetyl cysteine, also referred to as N-acetyl-L-cysteine, and abbreviated NAC, is a pharmaceutical drug and nutritional supplement used as a mucolytic agent in the treatment of chronic bronchitis and in the management of

acetaminophen (paracetamol) overdoses.

NAC is a potent thiol-containing antioxidant and is a non-antibiotic compound that possesses antimicrobial properties. NAC has been shown to effectively reduce biofilm formation in a variety of medically important gram-positive and gram-negative bacteria, including *Pseudomonas aeruginosa, Klebsiella pneumoniae, Staphylococcus epidermidis, Staphylococcus aureus,* and *Escherichia coli.* NAC has been reported to inhibit growth and eradicate biofilm formation of *Enterococcus faecalis,* which is frequently found in root-canal treated teeth. NAC has also been found to be effective in reducing extracellular polysaccharide production. In the case of mature biofilms, N-acetyl-L-cysteine has been reported as affecting growth, extracellular polysaccharide production, and bacterial biofilm formation on solid surfaces.

Polyphenols are a group of chemicals found in many fruits, vegetables, and other plants, such as berries, walnuts, olives, tea leaves and grapes. They are classified as antioxidants, meaning that they remove free radicals from the body, thereby preventing damage to cells and tissues. Polyphenols have been found to possess a variety of potential health benefits, including cancer prevention and reducing the risk of heart diseases.

Cranberry (*Vaccinium* species) is a berry producing plant found in North American bogs. The berry is edible and is eaten raw or used in the production of jams, pie fillings, and beverages. As with most similar berries, they are noted for antioxidant properties. The cranberry polyphenols and their use as anti-cariogenic and anti-periodontal disease agents are already known (Bonifait, L, Grenier D, J Can Dent Assoc 2010, 76; a130).

The present invention provides a novel combination of NAC, or any derivative thereof and polyphenols, specifically cranberry polyphenols. The invention further provides uses of this novel composition for treating and preventing all forms of periodontal disease.

Thus, one object of the invention is to provide solutions to the unmet need of patients suffering from periodontal diseases or undergoing dental or periodontal medical procedures by administration of a combination of NAC, or any derivative thereof, and polyphenol, as a preventive composition, or alternatively, as a therapeutic composition before or after a periodontal disease has developed.

Another object of the invention is the provision of compositions comprising a combination of NAC, or any derivative thereof, and at least one polyphenol, specifically polyphenols originated from cranberry extract.

A further object of the invention is the provision of a method of treating, preventing or delaying the onset of a periodontal disease by administering a combination of NAC, or any derivative thereof, and at least one polyphenol.

A still further object of the invention is the provision of the use of combinations of NAC, or any derivative thereof, and at least one polyphenol, in the preparation of a medicament for the treatment of a periodontal disease.

Further purposes and advantages of this invention will appear as the description proceeds.

## Summary of the Invention

According to a first aspect, the invention relates to a composition comprising a combination of N-acetyl cysteine (NAC), or any derivatives thereof, and at least one polyphenol.

According to one embodiment, the NAC comprised within the combined composition of the invention may be selected from the group consisting of: 2-Acetamido-3-sulfanylpropanoic; S-(2-(1-carboxy-2-methylpropyl)isoindole-1-yl)-N-acetylcysteine; N-acetylcysteine lysinate; S-phenyl-N-acetylcysteine; N-acetyl-S-(N-methylcarbamoyl)cysteine; N-acetyl-S-pentachloro-1,3-butadienylcysteine; adamantyl-N-acetylcystein; chitosan-N-acetylcysteine conjugate; G4-S-nitroso-N-acetylcysteine; 4-hydroxyestradiol-2-N-acetylcysteine; N-Acetylcysteinamide; 4-hydroxyestrone N-acetylcysteine; S-(1-(4'-methoxyphenyl)-2-hydroxypropyl)-N-acetylcysteine; S-(N,N-diethyldithiocarbamoyl)-N-acetylcysteine; 2,4-dinitrophenyl-S-(N-acetylcysteine); S-(6-purinyl)-N-acetylcysteine; S-(3-oxopropyl)-N-acetylcysteine; S-(2-carboxyethyl)-N-acetylcysteine; S-(3-hydroxy-3-carboxy-n-propyl)-N-acetylcysteine; N-acetylcysteine-6,7-dihydro-7-hydroxy-1-hydroxymethyl-5H-pyrrolizine; S-(2-(N(7)-guanyl)ethyl)-N-acetylcysteine; acetylcysteine(asparaginyl-alanyl-asparaginyl-proline)3; S-trichlorovinyl-N-acetylcysteine; S-(2-methylbenzyl)-N-acetylcysteine; S-1,2-dichlorovinyl-N-acetylcysteine; 2-(acetylcysteine)-N-isopropylacetanilide; S-nitroso-N-acetylcysteine; and N-acetylcysteine deacetylase. One NAC derivative of particular interest is 2-Acetamido-3-sulfanylpropanoic.

In yet another embodiment, the polyphenol comprised within the combined composition of the invention is a cranberry polyphenol. The polyphenol according to the present invention may be selected from the group consisting of: cyanidin 3-galactoside; cyanidin 3-glucoside; cyanidin 3-O-glucoside; cyanidin 3-arabinoside; cyanidin 3-O-alpha-L-arabinopryranoside; peonidin 3-galactoside; peonidin 3-glucoside; peonidin 3-arabinoside; myricetin 3-xyloside; myricetin 3-arabinoside; quercetin 3-galactoside; quercetin 3-xyloside; quercetin 3-arabinopyranoside; quercetin 3-arabinofuranoside; quercetin 3-rhamnoside; myricetin; methoxyquercetin 3-xyloside; quercetin 3-coumaroyl galactoside; quercetin; quercetin 3-benzoyl galactoside; petunidin 3-arabinoside; petunidin 3-O-alpha-L-arabinopyranoside; idaein; delphinidin 3-rhamnoside; peonidin 3-O-beta-D-galactopyranoside; oxycoccicyanin; malvidin 3-arabinoside, and any combination thereof.

The composition of the invention comprises at least one pharmaceutically acceptable carrier, diluent, excipient and/or additive.

The invention further provides a pharmaceutical composition for treating, preventing, ameliorating, reducing or delaying the onset of a periodontal disease. The pharmaceutical composition comprises as an active ingredient a therapeutically effective amount of a combination of N-acetyl cysteine, or any derivatives thereof, and at least one polyphenol. According to one specific embodiment, the pharmaceutical composition of the invention is particularly applicable for treating, preventing, ameliorating, reducing or delaying the onset of a periodontal disease selected from gingivitis, perimucositis, periodontitis, chronic periodontitis, aggressive periodontitis, periodontitis as a manifestation of systemic disease, necrotizing ulcerative periodontitis, abscesses of the periodontium, combined periodontic-endodontic lesions and peri-implantitis.
The pharmaceutical composition of the invention is an oral topical composition.
The invention further encompasses the use of a therapeutically effective amount of a combination of NAC, or any derivative thereof, and at least one polyphenol, in the preparation of a medicament for treating, preventing, ameliorating, reducing or delaying the onset of a periodontal disease.
The combination of the invention is adapted for oral topical application.
All the above and other characteristics and advantages of the invention will be further understood through the following illustrative and non-limitative description of embodiments thereof, with reference to the appended drawings.

## Brief Description of the Drawings

**Figure 1:** ***NAC inhibits growth and reduces biofilms of Aggregibcater actynomycetemcomitans, and is ineffective against Porphyromonas gingivalis***
The effects of NAC (compound A) at concentrations of 0% to 20% weight per volume (W/V) on growth and pre-formed biofilms of *Aggregibcater actynomycetemcomitans and Porphyromonas gingivalis* were measured in terms of Log10 reduction.
Abbreviations: Agg. B. (*Aggregibcater actynomycetemcomitans,* biofilm); Agg. G.(*Aggregibcater actynomycetemcomitans*, growth); Por. B. (*Porphyromonas gingivalis,* biofilm); Por. G. (*Porphyromonas gingivalis,* growth).
**Figure 2:** ***Cranberry polyphenols inhibit growth of Aggregibcater actynomycetemcomitans, and are ineffective against Porphyromonas gingivalis***
The effects of cranberry extract (compound B) at concentrations of 0% to 5% weight per volume (W/V) on growth of *Aggregibcater actynomycetemcomitans and Porphyromonas gingivalis* were measured in terms of Log10 reduction. Abbreviations: A. a. (*Aggregibcater actynomycetemcomitans*); P. gin. *(Porphyromonas gingivalis).*
**Figure 3:** ***Combination of NAC and cranberry polyphenols synergistically inhibits growth and reduces biofilms of Aggregibcater actynomycetemcomitans and Porphyromonas gingivalis***

The effects of a combination of 20% NAC (compound A) and cranberry extract (compound B) at concentrations of 0% to 5% weight per volume (W/V) on growth and pre-formed biofilms of *Aggregibcater actynomycetemcomitans and Porphyromonas gingivalis* were measured in terms of Log10 reduction. Abbreviations: Agg. B. (*Aggregibcater actynomycetemcomitans,* biofilm); Agg. G. (*Aggregibcater actynomycetemcomitans,* growth); Por. B. (*Porphyromonas gingivalis,* biofilm); Por. G. (*Porphyromonas gingivalis,* growth).

## Detailed Description of the Invention

The inventors of the present application have surprisingly found that administration of a combination of NAC and cranberry extract to the periodontal pockets of patients suffering from periodontitis and peri-implantitis showed significant improvement in terms of reduced dental plaque (i.e. biofilm) formation and inflammatory clinical signs in the area of the periodontal lesion.
According to one aspect, the present invention provides a pharmaceutical composition for use in treating and/or preventing periodontal diseases, as specified hereinbelow, comprising a combination of an effective amount of N-acetyl cysteine (NAC) and at least one polyphenol. The composition of the invention further comprises at least one pharmaceutically acceptable carrier, diluent, excipient and/or additive.
N-acetyl cysteine, also referred to as N-acetylcysteine or N-acetyl-L-cysteine is a derivative of the amino acid cysteine, wherein an acetyl group is attached to the nitrogen atom. The molecular formula of the compound is $C_5H_9NO_3S$, having the following structural formula I:

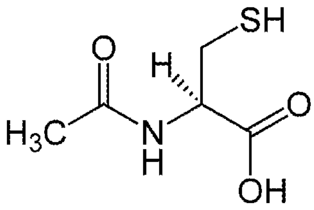

Formula I

According to one embodiment, the NAC compound comprised within the combined composition of the invention may be selected from the group consisting of:

2-Acetamido-3-sulfanylpropanoic acid;
S-(2-(1-carboxy-2-methylpropyl)isoindole-1-yl)-N-acetylcysteine;
N-acetylcysteine lysinate;
S-phenyl-N-acetylcysteine;
N-acetyl-S-(N-methylcarbamoyl)cysteine;
N-acetyl-S-pentachloro-1,3-butadienylcysteine;
adamantyl-N-acetylcystein;
chitosan-N-acetylcysteine conjugate;
G4-S-nitroso-N-acetylcysteine;
4-hydroxyestradiol-2-N-acetylcysteine;
N-Acetylcysteinamide;
4-hydroxyestrone N-acetylcysteine;
S-(1-(4'-methoxyphenyl)-2-hydroxypropyl)-N-acetylcysteine;
S-(N,N-diethyldithiocarbamoyl)-N-acetylcysteine;
2,4-dinitrophenyl-S-(N-acetylcysteine);
S-(6-purinyl)-N-acetylcysteine;
S-(3-oxopropyl)-N-acetylcysteine;
S-(2-carboxyethyl)-N-acetylcysteine;
S-(3-hydroxy-3-carboxy-n-propyl)-N-acetylcysteine;
N-acetylcysteine-6,7-dihydro-7-hydroxy-1-hydroxymethyl-5H-pyrrolizine;
S-(2-(N(7)-guanyl)ethyl)-N-acetylcysteine;
acetylcysteine(asparaginyl-alanyl-asparaginyl-proline)3;
S-trichlorovinyl-N-acetylcysteine;
S-(2-methylbenzyl)-N-acetylcysteine;
S-1,2-dichlorovinyl-N-acetylcysteine;
2-(acetylcysteine)-N-isopropylacetanilide;
S-nitroso-N-acetylcysteine; and
N-acetylcysteine deacetylase.

In yet another embodiment, the polyphenol comprised within the combined composition of the invention may be selected from the group consisting of: anthocyanins, flavonoids, phenol carboxylic acids, and proanthocyanidins. Specifically, the polyphenol is cranberry polyphenol. More specifically, the polyphenol of the invention is selected from the group consisting of:

cyanidin 3-galactoside;
cyanidin 3-glucoside;
cyanidin 3-O-glucoside;
cyanidin 3-arabinoside;
cyanidin 3-O-alpha-L-arabinopryranoside;
peonidin 3-galactoside;
peonidin 3-glucoside;
peonidin 3-arabinoside;
myricetin 3-xyloside;
myricetin 3-arabinoside;
quercetin 3-galactoside;
quercetin 3-xyloside;

quercetin 3-arabinopyranoside;
quercetin 3-arabinofuranoside;
quercetin 3-rhamnoside;
myricetin;
methoxyquercetin 3-xyloside;
quercetin 3-coumaroyl galactoside;
quercetin;
quercetin 3-benzoyl galactoside;
petunidin 3-arabinoside;
petunidin 3-O-alpha-L-arabinopyranoside;
idaein;
delphinidin 3-rhamnoside;
peonidin 3-O-beta-D-galactopyranoside;
oxycoccicyanin; and
malvidin 3-arabinoside.

It should be noted that the composition of the invention may comprise one or more of the above polyphenols in any combination.

According to one specific embodiment, the invention provides a pharmaceutical composition comprising 2-Acetamido-3-sulfanylpropanoic acid combined with cranberry extract, thus creating the NAC and cranberry polyphenol combined composition.

According to one embodiment, the combined composition of the invention may comprise NAC and cranberry extract, at any quantitative ratio of between about 1:1 to 100:1. It should be appreciated that any quantitative ratio of the combined compounds may be used. As a non-limiting example, the quantitative ratio used between the compounds of the invention is 1:4.

The compositions of the invention are suitable for oral administration, and are provided in a form adapted for topical application. The combined NAC and cranberry extract topical oral compositions can be administered from one or more times per day to one or more times per week, including once every other day. The combined NAC and cranberry extract compositions can be administered subgingivaly and have an inhibitory long standing effect on biofilm formation. The skilled artisan will appreciate that certain factors may influence the dosage and timing required to effectively treat a subject, including but not limited to the severity of the disease or disorder, previous treatments, the general health and/or age of the subject, and other diseases present.

Moreover, treatment of a subject with a therapeutically effective amount of the combined compounds can include a single treatment or, can include a series of few applications, in intervals of between 1 to 3 days.

The administration of the pharmaceutical combination of the invention results in a surprising beneficial effect, namely a synergistic therapeutic effect with regard to alleviating, delaying progression of or inhibiting a periodontal disease or peri-implantitis, compared with a mono-therapy applying only one of the pharmaceutically active ingredients used in the combination of the invention.

Accordingly, the invention provides a pharmaceutical composition comprising a quantity of a first active ingredient and a second active ingredient as previously described, which may be jointly therapeutically effective at targeting or preventing periodontal diseases. These first and second ingredients may be provided for administration in a fixed combination, i.e. in a single composition, which may be prepared in a manner known in the art, suitable for topical oral administration to mammals, including humans, in combination with one or more pharmaceutically acceptable carriers or diluents, especially suitable for topical oral application.

Currently available NAC formulations are distributed as solutions for inhalation, intravenous administration or oral use. However, these preparations are not suitable for topical implementation in the periodontal pocket in routine clinical applications. It should be noted that in order to achieve the desired clinical effect, a composition applied into the periodontal pocket has to be implicated for a minimal amount of time. Accordingly, aqueous topical oral compositions comprising NAC were found to be insufficient for administration into the periodontal pocket due to fast local leaking thereof from the pocket, as well as due to relatively rapid dilution of the composition by saliva.

Accordingly, one embodiment of the invention provides a composition in the form of a gel adapted for direct application into the periodontal pocket. The composition comprises an incipient matrix physically stabilizing the active ingredients of the composition, effectively confining the distribution thereof to the periodontal pocket and the area adjacent thereto for a desired period of time.

The topical oral composition according to the invention is directly inserted or injected into a periodontal pocket by a physician, e.g., a dentist. According to a specific embodiment, the composition is administered after dental or periodontal therapy such as scaling and root planning or deplaquing. As the periodontal pockets vary in size, the amount of the topical oral composition applied into a single pocket should be determined by the person conducting the procedure.

According to a specific embodiment, the composition of the invention is inserted into a single periodontal pocket in an amount of between about 0.01 to 1 ml, specifically, 0.1 ml. The active ingredients are included in the topical oral composition of the invention at a concentration of between about 5% to about 60% NAC, and between about 0.04% to about 30% cranberry extract by weight of the composition. More specifically, the active ingredients are included in concentrations of between about 15% to about 30% NAC, and between about 0.8% to about 10% cranberry extract, even more specifically, about 20% NAC and between about 2.5% and 5% cranberry extract, by weight of the final composition.

In another embodiment, the composition of the invention is provided in the form of a mouthwash, comprising the active ingredients in a suitable liquid carrier, for use in the prevention or treatment of periodontal diseases. This type of administration is especially beneficial for the prophylaxis or treatment of non-destructive periodontal disease, such as gingivitis, and as a maintenance treatment for periodontal diseases.

The oral composition of the present invention may also contain flavoring agents and/or breath-freshening agents, as well as sweeteners. Examples of flavoring agents which are used in the practice of the present invention include spearmint, peppermint, and menthol. The sweetener comprised in the mouthwash is a non-sugar synthetic sweetener.

In the present invention, an oral composition containing a combination of NAC and any derivative thereof, and cranberry extract is used in a method to prevent or treat dental-related diseases, such as a periodontal diseases, particularly gingivitis, peri-mucositis, periodontitis and peri-implantitis by administering the composition to the oral cavity of human or animal. The method is especially useful to prevent or treat dental inflammatory diseases such as gingivitis, peri-mucositis, periodontitis and peri-implantitis since the present combinations have superior and synergistic anti-bacterial and antiinflammatory efficacy.

The oral compositions of the invention may serve as useful adjuncts to mechanical debridement treatments, by providing profound inhibition of growth of the bacterial strains forming the biofilms. Accordingly, application of NAC and cranberry extract combination in the periodontal pocket as part of mechanical debridement procedures supports the recovery of the subject suffering from a periodontal disease and minimizes the need for repeated invasive periodontal treatments.

It should be noted that the protective and therapeutic synergistic effect of the combined composition of the invention is clearly demonstrated in the Examples and Figures, showing clear and unambiguous growth inhibition of two major bacterial strains present in dental biofilms, compared to the inhibitory effect of each one of the active materials alone.

Another aspect of the invention provides a combination of NAC, or any derivative thereof, and at least one polyphenol for use in the treatment of a dental-related disease, such as a periodontal disease. The invention also provides a combination of NAC, or any derivative thereof, and at least one polyphenol for use in preventing, ameliorating, reducing or delaying the onset of a periodontal disease. In certain embodiments, the combination comprises 2-Acetamido-3-sulfanylpropanoic and at least one cranberry polyphenol.

The combined compositions of the present invention are useful as both prophylactic and therapeutic treatments for periodontal diseases selected from the group consisting of gingivitis, peri-mucositis, periodontitis, chronic periodontitis, aggressive periodontitis, periodontitis as a manifestation of systemic disease, necrotizing ulcerative periodontitis, abscesses of the periodontium, combined periodontic-endodontic lesions and peri-implantitis.

In a further aspect the invention provides the use of a combination of NAC, or any derivative thereof, and at least one polyphenol in the preparation of a medicament for treating a dental-related disease, specifically a periodontal disease.

The present invention relates to the treatment of subjects, or patients, in need thereof. By "patient" or "subject in need" it is meant any mammal for which administration of the combined composition of the invention is desired, in order to prevent, overcome or slow down such condition.

The terms "treatment", "prevention" and "prophylaxis" refer to the complete range of therapeutically positive effects of administrating to a subject including inhibition, reduction of, alleviation of, and relief from, a periodontal disease, specifically, gingivitis, peri mucositis, severe periodontitis and peri-implantitis. More specifically, treatment or prevention includes the prevention or postponement of development of the disease, prevention or postponement of development of symptoms and/or a reduction in the severity of such symptoms that will or are expected to develop. These further include ameliorating existing symptoms, preventing additional symptoms and ameliorating or preventing the underlying causes of symptoms.

It should be noted that particularly in case of human subjects, administration of the compositions of the invention to the patient includes both self-administration and administration to the patient by another person.

The term "inhibition" as referred to herein, relates to the retardation, attenuation, retraining or reduction of a process. More specifically, according to certain embodiments, the combined compositions and also any of the methods of the invention specifically inhibit biofilm formation by about 50% to 100%, more specifically about 80% to 99.9%, as compared to untreated subjects suffering from periodontitis or peri-implantitis.

With regards to the above, it is to be understood that, where provided, percentage values such as, for example,

10%, 50%, 120%, 500%, etc., are interchangeable with "fold change" values, i.e., 0.1, 0.5, 1.2, 5, etc., respectively.

To provide a "preventive treatment" or "prophylactic treatment" is acting in a protective manner, to defend against or prevent something, especially a condition or disease.

The term "destructive periodontal disease" as used herein refers to the following categories of periodontal diseases and conditions: gingivitis, peri-mucositis, chronic periodontitis, aggressive periodontitis, periodontitis as a manifestation of systemic disease, necrotizing ulcerative periodontitis, abscesses of the periodontium and combined periodontic-endodontic lesions; as standardized in the 1999 classification system for periodontal diseases and conditions.

The term "periodontal disease" as used herein also includes an inflammatory process with loss of supporting bone in the tissues surrounding functioning dental implants, which is defined as peri-implantitis.

As used herein, "disease", "disorder", "condition" and the like, as they relate to a subject's health, are used interchangeably and have meanings ascribed to each and all of such terms.

The term "pharmaceutical composition" refers to an active compound in any form suitable for effective administration to a subject, e.g., a mixture of the compound and at least one pharmaceutically acceptable carrier.

The term "therapeutically effective amount" is intended to mean that amount of a drug or pharmaceutical agent that will elicit the biological or medical response of a tissue, animal or human that is being sought by a researcher, veterinarian, medical doctor, dentist, periodontist or other clinician, or by the subject himself.

As used herein, a "pharmaceutically acceptable carrier" means a carrier or diluent that does not cause significant irritation to a subject and does not abrogate the biological activity and properties of the administered combination of compounds.

A "pharmaceutically acceptable excipient" means an inert substance added to a pharmaceutical composition to further facilitate administration of the combination of compounds. Examples, without limitation, of excipients include calcium carbonate, calcium phosphate, various sugars and types of starch, cellulose derivatives, gelatin, vegetable oils and polyethylene glycols.

The present invention therefore particularly relates to additive and synergistic combinations of NAC and cranberry extract polyphenols. Those additive and synergistic combinations are useful in treating subjects suffering from periodontal diseases, for example, gingivitis, peri-mucositis periodontitis and peri-implantitis. The synergistic and additive compositions of the invention may also be used for the treatment of subjects exhibiting symptoms or signs of such disorders.

By synergic combination is meant that the effect of both NAC and cranberry extract polyphenols is greater than the sum of the therapeutic effects of administration of any of these compounds separately, as a sole treatment.

The following examples, which further describe the invention, are offered by way of illustration and are not intended to limit the invention in any manner.

## Examples

### EXAMPLE 1

***Extraction of Cranberry Juice Fractions and Analysis of the Fractions by Mass Spectroscopy***

Preparation of Cranberry Juice Extract

Four cans of frozen concentrated cranberry juice (Ocean Spray Cranberries, Inc., Lakeville-Middleboro, MA) were purchased from Superstore and thawed. Dialysis tubing lengths (approximately 67 cm each, VWR product code 25225-260, 45 mm flat width, MWCO 12000-14000) were soaked in room temperature dd$H_2O$ in a bucket. A total of 25 tubes were prepared. Large containers were cleaned and rinsed with dd$H_2O$ three times, and then placed in the cold room and filled with cold dd$H_2O$. The bottom of each tube was clamped. 1000 mL of concentrated cranberry solution was divided between the 25 tubes (40 mL/tube). All the tubes were soaked in the cold water in two large containers. Enough cold water was used to cover the tubes completely in the containers. The dialysis was performed for a total of 5 days. The cold water was changed once a day during the dialysis. On the first day, the cold water became deep pink. After the water was changed additional times, the color of the water collected became a paler pink.

At the fifth day, the non-dialyzable material (NDM) was transferred from the dialysis tubing to HDPE bottles. 35 HDPE bottles were filled with a total of 3.8L of NDM-containing solution. All the bottles were placed in the freezer at -80°C. Once all the solution had been frozen at -80°C overnight, the bottles were transferred to trays, the lids were removed, Kimwipes held in place with rubber bands were used to cover the mouths of the bottles, and the trays were placed in a pilot-scale freeze dryer at Alberta Innovates Technology Futures (AITF). After 10 days freeze drying, 1.92 g of the crystalline red-purple solid cranberry extract was collected from the 35 bottles. 0.053 g of the extract was analyzed by electrospray mass spectroscopy.

Chemical Characterization of Cranberry (*Vaccinium*) Extract

20 μL of the cranberry extract were used to chromatographically characterize the materials using high performance liquid chromatography (HPLC) coupled to diode array absorbance (DAD) and positive mode electrospray mass spectrometric (LC/MS) detection. Negative mode electrospray mass spectrometric (LC/MS) detection was also performed, providing additional information.

Upon running the samples through the chromatographic system, a number of peaks were observed. Several peaks with some absorbance in the red region (456 nm) were observed. Mass spectrometric signals at 419, 433, 449, and 463 suggested that these were anthocyanidin sugar conjugates (anthocyanins) previously described in *Vaccinium* and other plants in the literature. A number of relatively well separated peaks with strong absorbance at 254 nm were also observed. Mass spectrometric signals suggested that these might be quercetin conjugates as fragments with mass 302 were observed.

The identities of some of the compounds in the cranberry extract are provided in Table 1.

**Table 1:** Possible identities and molecular formulae of some anthocyanins and flavones found in the cranberry extract.

| Molecular weight | Chemical formula | Possible Identity |
|---|---|---|
| 419<br>419 | $C_{20}H_{19}O_{10}$<br>$C_{20}H_{19}O_{10}$ | Cyanidin 3-arabinoside<br>Cyanidin 3-O-alpha-L-arabinopryranoside |
| 433 | $C_{21}H_{21}O_{10}$ | Peonidin 3-arabinoside |
| 449<br>449<br>449<br>449<br>449 | $C_{21}H_{21}O_{11}$<br>$C_{21}H_{21}O_{11}$<br>$C_{21}H_{21}O_{11}$<br>$C_{21}H_{21}O_{11}$<br>$Cr_{21}H_{21}O_{11}$ | Cyanidin 3-O-glucoside<br>Petunidin 3-arabinoside<br>Petunidin 3-O-alpha-L-arabinopyranoside<br>Idaein<br>Delphinidin 3-rhamnoside |
| 463<br>463<br>463 | $C_{22}H_{23}O_{11}$<br>$C_{22}H_{23}O_{11}$<br>$C_{22}H_{23}O_{11}$ | Peonidin 3-O-beta-D-galactopyranoside<br>Oxycoccicyanin<br>Malvidin 3-arabinoside |
| 484 (fragment 318) -ve mode | $C_{30}H_{28}O_6$ | Epigallocatechin 3-O-caffeate |
| 468 (fragment 302) -ve mode | $C_{24}H_{20}O_{10}$ | Possible quercetin conjugate (condensation with $C_{10}H_{16}O_3$) |

The above results verify that the samples examined were obtained from *Vaccinium* Cranberry.

**EXAMPLE 2**

***Antimicrobial Activity Evaluation of NAC, Cranberry Extract, and Combinations Thereof Using Dental Biofilms of Selected Bacterial Strains***

*Materials and Experimental procedures*

Tested Biocides

- NAC, also referred to herein as DentalMed Pharm product Mucomyst, examined at concentrations of 0.015%-20% μg/mL, designated as compound A.
- Cranberry *Vaccinium* extract, tested at concentrations of 0.04%-5% μg/mL, designated as compound B.
- Combinations of NAC (20% μg/mL) and Cranberry *Vaccinium* extract (0.04%-5%), also referred to herein as compounds A+B.

HA MBEC™ P&G plate

Sterile MBEC™ P&G plates coated with Hydroxyapatite were used for biofilm growth. The plates consist of plastic lids with 96 protruding polystyrene pegs that have each been coated with hydroxyapatite and a corresponding 96 well base into which the inoculum is placed. The bacterial inoculum for each plate is 150 μL in each well (Innovotech Inc., Edmonton, Alberta, Canada).

Microorganisms

1. *Porphyromonas gingivalis* (source: ATCC 33277).
2. *Aggregibcater actynomycetemcomitans* (source: ATCC 43717).

*The experimental process for high-throughput antimicrobial susceptibility testing using the MBEC™ P&G assay*

Culture/Inoculum Preparation

Using a cryogenic stock (at -70ºC), a first sub-culture of the bacterial organisms listed above was streaked out on an organism specific agar (OSA). The bacterium was incubated at appropriate growth conditions for 24-48 hours and the plate was stored wrapped in parafilm at 4ºC for the aerobic strain (*Aggregibcater actynomycetemcomitans*) and 21°C in the anaerobic chamber for anaerobic strain (*Porphyromonas gingivalis*). From the first sub-culture, a second sub-culture was streaked out on OSA. It was incubated at appropriate growth conditions for 24-48 hours. The second sub-culture was used within 24 hours starting from the time it was first removed from incubation. Using the second sub-culture, an isolated colony was aseptically removed from the OSA plate and inoculated into 100 mL of sterile bacterial liquid growth broth. The latter was incubated at 150 rpm at appropriate growth conditions for 24-24 hour. This yielded a viable bacterial density of approximately $10^9$ CFU/mL and was checked by serial dilution and plating. The inoculum was adjusted to an approximate cell density of $10^6$ CFU/mL by diluting in organism specific broth (OSB). The diluted organism was vortexed for approximately 10 seconds to achieve uniform mixing of the organism. One sample (100 μL) of the diluted organism was used for an inoculum check by serially diluting and spot plating on OSA in triplicate. It should be noted that for the anaerobic microorganism, growth, sonication, serial dilution, transfer and recovery of the organism was performed under anaerobic conditions (5% $H_2$, 10% $CO_2$, 85% $N_2$).

*Pre-formed Biofilm Testing*

Growing the Biofilms

Each bacterial over-night culture was diluted 1,000x in its specific OSM. Using a micro pipette, 150 μL of inoculum was added to the appropriate wells of a 96 well plate, except for wells that served as sterility controls (SC). Sterility control wells received sterile OSB. The HA MBEC™ P&G lid was then inserted into the 96-well bottom. It should be noted that the volume of inoculum used in this step has been calibrated such that the biofilm covers a surface area that is immersed, entirely, by the volume of antimicrobials used in the challenge plate set up. The device was placed on the gyrorotary shaker in a humidified incubator and incubated at 110 rpm at the appropriate growth conditions. *Porphyromonas gingivalis* was incubated for 24 hours, and *Aggregibcater actynomycetemcomitans* was incubated for 48 hours. A triplicate sample of the inoculum was serially diluted (ten-fold). The latter served as controls used to verify the starting cell number in the inoculum. The serial 10-fold dilutions of the inoculum from $10^{-7}$ to $10^0$ were spot plated on an appropriately labeled series of agar plates. The spot plates were incubated for an appropriate period of time and scored for growth.

Biofilm growth check

The biofilm growth check (GCh) pegs were broken off from the challenge plate with flamed pliers. Each peg was placed into 200 μL of neutralizer in row A of serial dilution plate. The plates were sonicated for 30 minutes. Then serial dilution and spot plating on OSA was performed, which served as a biofilm growth check.

Preparation of Challenge Plates

Biofilms were tested using the specified contact times. Callenge plates were used for testing the killing activity

of the tested biocidic compounds on formed biofilm and other plates to test the inhibitory effect of biofilm formation. SC wells served as sterility controls for each experiment. One challenge plate was used per growth condition and challenge time. GC is the growth control. GCh is the biofilm Growth Check.

Using a sterile 96-well microtitre plate the following was done aseptically to set up the above challenge plates. 200 μL of sterile OSM was added to wells that served as the device sterility control for the media and for biofilm growth checks. 200 μL of sterile neutralizer was added to wells that served as the neutralizer toxicity control. 100 μL of the neutralizer and 100 μL of each of the tested compounds or combinations were added to wells serving as neutralizer affectivity control. 200 μL of biocide stock solution was added to wells which served as the biocide diluent sterility control. Serial dilutions of the tested compounds and combinations in the diluents (OSM) were performed. Each freshly prepared challenge plate was aseptically covered and left to stand at room temperature for 30 minutes to equilibrate prior to use.

Antimicrobial Challenge of pre-formed Biofilm

Planktonic cells were rinsed from the biofilm that formed on the lid of the MBEC device by dipping the lid into saline for 1-2 minutes. The lid was transferred to the challenge plate and incubated at 35 ± 2°C for 24 hours in the anaerobic/aerophilic chamber. Neutralizer plate(s) of the neutralizer (200 μL per well) were prepared. After 24 hours, the MBEC™ lid was transferred to the neutralizer plate. It was allowed to stand for 30 minutes to equilibrate. The plate was transferred to the sonicator and sonicated on high for 30 minutes to dislodge surviving biofilm. The plates were then placed in a dry stainless steel insert tray which sits in the water of the sonicator. The vibrations created in the water by the sonicator transferred through the insert tray to actively sonicate the contents of the 96 well recovery plates.

*$LOG_{10}$ Reduction*

Following sonication of all plates, 100 μL was placed from each well of the MBECTM plate into the first 12 empty wells of the first row of a 96 well-micro titer plate. 180 μL of sterile 0.9% saline were placed in the remaining rows. A Serial dilution ($10^0$-$10^{-7}$) was performed by moving 20 μL down each of the 8 rows. 10 μL were removed from each well and spot plated on prepared OSA plates. Plates were incubated at 37±1ºC and counted after approximately 24-48 hours of incubation. The data obtained was evaluated as Log10 CFU/peg. 100 μL of the sterile neutralizer were placed in each well of the recovery plate to top up the volume back to 200 μL.

*Cell Enumeration:*

The appropriate number of colonies was counted according to the plating method used. The arithmetic mean of the colonies counted on the plates was calculated.

The log density for one peg was calculated as follows:

$$LOG_{10}\,(CFU/peg) = LOG_{10}[(X/B)\,(D)]$$

where:

*X* = mean CFU;
*B* = volume plated (0.02 mL); and
*D* = dilution.

The overall biofilm accumulation was calculated by calculating the mean of the log densities calculated.

The $LOG_{10}$ reduction for each dilution was calculated as follows:

$$LOG_{10}\ Reduction = Mean\ LOG_{10}\ Growth\ Control - Mean\ LOG_{10}\ Test.$$

*Determination of Planktonic MIC*

The challenge plate was incubated at 37°C for 24 hours and read visually to determine MIC values. The MIC (minimum inhibitory concentration) for the compound was determined for each organism shed from the biofilm during the challenge incubation. The MIC was defined as the minimum concentration that inhibits growth of the organism.

*Determination of Planktonic Minimum Bactericidal Concentration (MBC)*

After the specified contact time (24 hours), 20 μL from each well of the challenge plate were removed and placed into the corresponding wells of a fresh 96 well Nunc plate containing 180 μL OSM. The plate was incubated at 37°C for 24 hours. MBC results, representing the lowest concentration of the test compound required to kill the bacterium, were determined following the 24 hour incubation by +/- growth.

*Determination of Biofilm MBEC*

MBEC is defined as the minimum concentration of test compound that inhibits growth of the biofilm. The recovery plate prepared according to the description above was incubated at 37°C for 24 hours and read visually to determine MBEC values. A microtiter plate reader was used to obtain optical density measurements at 630 nm ($OD_{630}$). Clear wells ($OD_{630}$ < 0.1) ware evidence of biofilm eradication.

Results

**NAC**

**Table 2:** Log Reduction Summary for Compound A

| | **A.a.** | | ***P. gingivalis*** | |
|---|---|---|---|---|
| **Compound A** | **Biofilm** | **Growth Inhibition** | **Biofilm** | **Growth Inhibition** |
| 20% | 3.25 | 6.34 | 0.13 | 0.36 |
| 10% | 3.02 | 6.34 | -0.17 | -0.71 |
| 5% | 0.39 | 2.05 | -0.18 | -0.19 |
| 2.50% | -0.01 | 1.13 | 0.13 | 0.11 |
| 1.25% | 0.36 | 1.04 | -0.18 | 0.44 |
| 0.62% | 0.23 | 0.74 | -0.07 | 0.35 |
| 0.31% | -0.02 | 0.28 | 0.07 | -0.05 |
| 0.15% | 0.51 | 0.24 | 0.11 | -0.89 |

The above results are graphically presented in Fig. 1. Compound A (NAC) was effective at inhibiting growth of *Aggregibcater actynomycetemcomitans* (A.a.) only at 20% and 10% of the stock concentration. Biofilms had a >3 log reduction at 20% and 10% of the stock concentration. In the case of *Porphyromonas gingivalis* (*P. gingivalis*), Compound A was ineffective at inhibiting growth and reducing preformed biofilm at all tested concentrations.

**Table 3:** MIC, MBC and MBEC Cut-Off Values

| **Compound A** | | |
|---|---|---|
| **Cut-off** | ***A.a.*** | ***P. gingivalis*** |
| MIC | 10% | >20% |
| MBC | >20% | >20% |
| MBEC | >20% | >20% |

The MIC, MBC and MBEC data for Compound A indicates that the compound had a greater growth inhibition effect than a biofilm inhibition effect on the tested *Aggregibacter actynomycetemcomitans* (A.a.) No inhibition or biofilm reduction of the *Porphyromonas gingivalis* (*P. gingivalis*) was observed at the test concentrations.

**Cranberry Extract**

**Table 4:** Log Reduction Summary for Compound B

| Compound B Con. | *A.a.* | *P. gingivalis* |
|---|---|---|
| 5.00% | 6.46 | 0.05 |
| 2.50% | 1.76 | 1.05 |
| 1.25% | 1.27 | 0.14 |
| 0.63% | 0.63 | 0.11 |
| 0.31% | 0.92 | -0.10 |
| 0.16% | 0.74 | 0.61 |
| 0.08% | 0.96 | 0.33 |
| 0.04% | 0.53 | -0.63 |

The above results are graphically presented in Fig. 2. Compound B had activity against *A. actynomycetemcomitans* (*A.a.*) at 5.0% of the stock concentration with a log reduction of 6.46. Some activity was seen at the 2.5% and 1.25% with 1.76 and 1.25 log reductions respectively. Furthermore, Compound B on its own was not effective against *P. gingivalis.*

**Combination of NAC and Cranberry Extract**

**Table 5:** Log reduction summary for compounds A+B

| | | *A.a.* | | *P. gingivalis* | |
|---|---|---|---|---|---|
| A | B | **Biofilm** | **Growth Inhibition** | **Biofilm** | **Growth Inhibition** |
| 20.0% | 5.00% | 6.99 | 6.50 | 4.15 | 4.10 |
| 20.0% | 2.50% | 6.99 | 6.50 | 2.56 | 5.42 |
| 20.0% | 1.25% | 6.55 | 6.50 | 2.90 | 5.42 |
| 20.0% | 0.63% | 4.06 | 6.50 | 1.28 | 3.58 |
| 20.0% | 0.31% | 3.97 | 6.50 | 1.66 | 2.67 |
| 20.0% | 0.16% | 3.00 | 6.50 | 0.33 | 0.76 |
| 20.0% | 0.08% | 2.83 | 6.50 | 1.15 | 1.02 |
| 20.0% | 0.04% | 3.13 | 5.91 | 1.50 | 0.17 |

The above results are graphically presented in Fig. 3. In the case of *Aggregatibacter actynomycetemcomitans* (*A.a.*), the combination of compounds A (NAC) and B (cranberry extract) had complete growth inhibition at concentrations of 0.08% or higher of compound B and a >5 log reduction at 0.04% compound B with 20% compound A.

In the case of *P. gingivalis* the combination was effective at inhibiting growth at >3 log reduction with compound B at concentrations of 0.63% or higher with 20% compound A. Pre-formed biofilms were more resistant and had a >3 log reduction at 5.0% compound B with 20% compound A.

**Table 6:** MIC, MBC and MBEC Cut-Off Values

| Cut-off | *A.a* | *P. gingivalis* |
|---|---|---|
| MIC | B0.1% | B2.5% |
| MBC | B2.5% | B5.0% |

(continued)

| Cut-off | *A.a* | *P. gingivalis* |
|---|---|---|
| MBEC | B5.0% | B5.0% |

The MIC, MBC and MBEC data obtained after treatment with the combination of compounds A and B indicates that the combination had a greater growth inhibition effect than a biofilm inhibition effect on the tested bacterial strains.

Thus, the combination according to the invention clearly led to a more prominent decrease in both inhibiting bacterial strains constituting dental biofilms and in reducing the development of existing biofilms, compared to the effect of each of the active ingredients of the combination alone.

In summary, the present invention discloses a synergistic combination of NAC and cranberry polyphenols. The results presented hereinabove demonstrate the feasibility and beneficial effect of using a combination of NAC and cranberry polyphenols for topical oral administration in order to successfully prevent or ameliorate periodontal diseases, specifically gingivitis, peri-mucositis, periodontitis and peri-implantitis.

## Claims

1. A composition for use in treating, preventing, ameliorating, reducing or delaying periodontal disease, said composition comprising a synergistic combination of N-acetyl cysteine (NAC) and at least one cranberry polyphenol and a pharmaceutically acceptable carrier for topical oral application of said combination.

2. The composition for use according to claim 1, wherein said NAC is selected from the group consisting of 2-Acetamido-3-sulfanylpropanoic; S-(2-(1-carboxy 2-methylpropylisoindole-1-yl)-N-acetylcysteine; N-acetylcysteine lysinate; S-phenyl-N-acetylcysteine; N-acetyl-S-(N-methylcarbamoyl)cysteine; N-acetyl-S-pentachloro-1,3-butadienylcysteine; adamantyl-N-acetylcysteine; chitosan-N-acetylcysteine conjugate; G4-S-nitroso-N-acetylcysteine; 4-hydroxyestradiol-2-N-acetylcysteine; N-Acetylcysteinamide; 4-hydroxyestrone N-acetylcysteine; S-(1-(4'-methoxyphenyl)-2-hydroxypropyl)-N-acetylcysteine; S-(N,N-diethyldithiocarbamoyl)-N-acetylcysteine; 2,4-dinitrophenyl-S-(N-acetylcysteine); S-(6-purinyl)-N-acetylcysteine; S-(3-oxopropyl)-N-acetylcysteine; S-(2-carboxyethyl)-N-acetylcysteine; S-(3-hydroxy-3-carboxy-n-propyl)-N-acetylcysteine; N-acetylcysteine-6,7-dihydro-7-hydroxy-1-hydroxymethyl-5H-pyrrolizine; S-(2-(N(7)-guanyl)ethyl)-N-acetylcysteine; acetylcysteine(asparaginyl-alanyl-asparaginyl-proline)S; S-trichlorovinyl-N-acetylcysteine; S-(2-methylbenzyl)-N-acetylcysteine; S-1,2-dichlorovinyl-N-acetylcysteine; 2-(acetylcysteine)-N-isopropylacetanilide; S-nitroso-N-acetylcysteine; and N-acetylcysteine deacetylase.

3. The composition for use according to claim 2, wherein said NAC is 2-Acetamido-3-sulfanylpropanoic.

4. The composition for use according to claim 1, wherein said polyphenol is selected from the group consisting of: cyanidin 3-galactoside; cyanidin 3-glucoside; cyanidin s-O-glucoside; cyanidin 3-arabinoside; cyanidin 3-O-alpha-L-arabinopyranoside; peonidin 3-galactoside; peonidin 3-glucoside; peonidin 3-arabinoside; myricetin 3-xyloside; myricetin 3-arabinoside; quercetin 3-galactoside; quercetin 3-xyloside; quercetin 3-arabinopyranoside; quercetin 3-arabinofuranoside; quercetin 3-rhamnoside; myricetin; methoxyquercetin 3-xyloside; quercetin 3-coumaroyl galactoside; quercetin; quercetin 3-benzoyl galactoside; petunidin 3-arabinoside; petunidin 3-O-alpha-L-arabinopyranoside; idaein; delphinidin 3-rhamnoside; peonidin 3-O-beta-D-galactopyranoside; oxycoccicyanin; malvidin 3-arabinoside, and any combination thereof.

5. The composition for use according to claim 1, in the form of a gel.

6. The composition for use according to claim 1, in the form of a mouthwash.

7. The composition for use according to any preceding claim, wherein said periodontal disease is selected from any one of gingivitis, peri-mucositis, periodontitis, chronic periodontitis, aggressive periodontitis, periodontitis as a manifestation of systemic disease, necrotizing ulcerative periodontitis, abscesses of the periodontium, combined periodontic-endodontic lesions and peri-implantitis.

8. The composition for use according to any preceding claim, in a form suitable for subgingival administration.

9. The composition for use according to claim 6, wherein said carrier is a liquid carrier.

10. The composition for use according to any preceding claim comprising between 5% to 60% NAC and between 0.04% to 30% cranberry extract by weight of the composition.

11. The composition for use according to claim 10, wherein said combination comprises 20% NAC and between 2.5% and 5% cranberry extract by weight of the final composition.

12. A composition comprising a synergistic combination of N-acetyl cysteine (NAC) and at least one cranberry polyphenol and a pharmaceutically acceptable carrier for topical oral application of said combination, wherein said combination comprises between 5% and 60% NAC and between 0.04% and 30% cranberry extract by weight of the final composition.

13. A composition according to claim 12 wherein said combination comprises between 5% and 20% NAC and between 0.04% and 5% cranberry extract by weight of the final composition.

14. A composition according to claim 12 wherein said combination comprises between 15% and 30% NAC and between 0.8% to 10% cranberry extract by weight of the final composition.

**Patentansprüche**

1. Zusammensetzung zur Verwendung bei der Behandlung, Vorbeugung, Linderung, Verminderung oder Verzögerung von periodontaler Krankheit, wobei die Zusammensetzung eine synergistische Kombination aus N-Acetylcystein (NAC) und mindestens einem Moosbeerpolyphenol und einen pharmazeutisch akzeptablen Träger für die topische orale Anwendung der Kombination umfasst.

2. Zusammensetzung zur Verwendung nach Anspruch 1, wobei das NAC ausgewählt ist aus der Gruppe bestehend aus 2-Acetamido-3-sulfanylpropionsäure; S-(2-(1-Carboxy-2-methylpropylisoindol-1-yl)-N-acetylcystein; N-Acetylcysteinlysinat; S-Phenyl-N-acetylcystein; N-Acetyl-S-(N-methylcarbamoyl)cystein; N-Acetyl-S-pentachlor-1,3-butadienylcystein; Adamantyl-N-acetylcystein; Chitosan-N-acetylcystein-Konjugat; G4-S-Nitroso-N-acetylcystein; 4-Hydroxyöstradiol-2-N-acetylcystein; N-Acetylcysteinamid; 4-Hydroxyöstron-N-acetylcystein; S-(1-(4'-Methoxyphenyl)-2-hydroxypropyl)-N-acetylcystein; S-(N,N-Diethyldithiocarbamoyl)-N-acetylcystein; 2,4-Dinitrophenyl-S-(N-acetylcystein); S-(6-Purinyl)-N-acetylcystein; S-(3-Oxopropyl)-N-acetylcystein; S-(2-Carboxyethyl)-N-acetylcystein; S-(3-Hydroxy-3-carboxy-n-propyl)-N-acetylcystein; N-Acetylcystein-6,7-dihydro-7-hydroxy-1-hydroxymethyl-5H-pyrrolizin; S-(2-(N(7)-Guanyl)ethyl)-N-acetylcystein; Acetylcystein(asparaginyl-alanyl-asparaginyl-prolin)S; S-Trichlorvinyl-N-acetylcystein; S-(2-Methylbenzyl)-N-acetylcystein; S-1,2-Dichlorvinyl-N-acetylcystein; 2-(Acetylcystein)-N-isopropylacetanilid; S-Nitroso-N-acetylcystein und N-Acetylcysteindeacetylase.

3. Zusammensetzung zur Verwendung nach Anspruch 2, wobei das NAC 2-Acetamido-3-sulfanylpropionsäure ist.

4. Zusammensetzung zur Verwendung nach Anspruch 1, wobei das Polyphenol ausgewählt ist aus der Gruppe bestehend aus: Cyanidin-3-galactosid; Cyanidin-3-glucosid; Cyanidin-s-O-glucosid; Cyanidin-3-arabinosid; Cyanidin-3-O-alpha-L-arabinopyranosid; Peonidin-3-galactosid; Peonidin-3-glucosid; Peonidin-3-arabinosid; Myricetin-3-xylosid; Myricetin-3-arabinosid; Quercetin-3-galactosid; Quercetin-3-xylosid; Quercetin-3-arabinopyranosid; Quercetin-3-arabinofuranosid; Quercetin-3-rhamnosid; Myricetin; Methoxyquercetin-3-xylosid; Quercetin-3-coumaroylgalactosid; Quercetin; Quercetin-3-benzoylgalactosid; Petunidin-3-arabinosid; Petunidin-3-O-alpha-L-arabinopyranosid; Idaein; Delphinidin-3-rhamnosid; Peonidin-3-O-beta-D-ga!actopyranosid; Oxycoccicyanin; Malvidin-3-arabinosid und irgendeiner Kombination davon.

5. Zusammensetzung zur Verwendung nach Anspruch 1 in Form eines Gels.

6. Zusammensetzung zur Verwendung nach Anspruch 1 in Form eines Mundwassers.

7. Zusammensetzung zur Verwendung nach einem der vorstehenden Ansprüche, wobei die periodontale Krankheit ausgewählt ist aus einem von Gingivitis, Perimukositis, Periodontitis, chronischer Periodontitis, aggressiver Periodontitis, Periodontitis als eine Manifestation einer systemischen Erkrankung, nekrotisierender ulzerierender Periodontitis, Abszessen des Periodontiums, kombinierten periodontischenendodontischen Läsionen und Periimplantitis.

8. Zusammensetzung zur Verwendung nach einem der vorstehenden Ansprüche in einer Form, die zur subgingivalen

Verabreichung geeignet ist.

**9.** Zusammensetzung zur Verwendung nach Anspruch 6, wobei der Träger ein flüssiger Träger ist.

**10.** Zusammensetzung zur Verwendung nach einem der vorstehenden Ansprüche, umfassend zwischen 5 % und 60 % NAC und zwischen 0,04 % und 30 % Moosbeerextrakt, bezogen auf das Gewicht der Zusammensetzung.

**11.** Zusammensetzung zur Verwendung nach Anspruch 10, wobei die Kombination 20 % NAC und zwischen 2,5 % und 5 % Moosbeerextrakt, bezogen auf das Gewicht der Endzusammensetzung, umfasst.

**12.** Zusammensetzung, umfassend eine synergistische Kombination aus N-Acetylcystein (NAC) und mindestens einem Moosbeerpolyphenol und einen pharmazeutisch akzeptablen Träger für die topische orale Anwendung der Kombination, wobei die Kombination zwischen 5 % und 60 % NAC und zwischen 0,04 % und 30 % Moosbeerextrakt, bezogen auf das Gewicht der Endzusammensetzung, umfasst.

**13.** Zusammensetzung nach Anspruch 12, wobei die Kombination zwischen 5 % und 20 % NAC und zwischen 0,04 % und 5 % Moosbeerextrakt, bezogen auf das Gewicht der Endzusammensetzung, umfasst.

**14.** Zusammensetzung nach Anspruch 12, wobei die Kombination zwischen 15 % und 30 % NAC und zwischen 0,8 % und 10 % Moosbeerextrakt, bezogen auf das Gewicht der Endzusammensetzung, umfasst.

## Revendications

**1.** Composition à utiliser dans le traitement, la prévention, l'amélioration, la réduction ou le ralentissement d'une maladie parodontique, ladite composition comprenant une combinaison synergique de N-acétylcystéine (NAC) et d'au moins un polyphénol de canneberge et un vecteur pharmaceutiquement acceptable pour une application orale topique de ladite combinaison.

**2.** Composition à utiliser selon la revendication 1, dans laquelle ladite NAC est sélectionnée à partir du groupe constitué par du 2-acétamido-3-sulfanylpropanoïque ; du S-(2-(1-carboxy 2-méthylpropylisoindole-1-yl)-N-acétylcystéine ; du lysinate de N-acétylcystéine ; de la S-phényl-N-acétylcystéine ; de la N-acétyl-S-(N-méthylcarbamoyl)cystéine ; de la N-acétyl-S-pentachloro-1,3-butadiénylcystéine ; de la adamantyl-N-acétylcystéine ; un conjugué de chitosan-N-acétylcystéine ; de la G4-S-nitroso-N-acétylcystéine ; de la 4-hydroxyestradiol-2-N-acétylcystéine ; du N-acétylcystéinamide ; de la 4-hydroxyestrone N-acétylcystéine ; de la S-(1-(4'-méthoxyphényl)-2-hydroxypropyl)-N-acétylcystéine ; de la S-(N,N-diéthyldithiocarbamoyl)-N-acétylcystéine ; de la 2,4-dinitrophényl-S-(N-acétylcystéine) ; de la S-(6-purinyle)-N-acétylcystéine ; de la S-(3-oxopropyl)-N-acétylcystéine ; de la S-(2-carboxyéthyl)-N-acétylcystéine ; de la S-(3-hydroxy-3-carboxy-n-propyl)-N-acétylcystéine ; de la N-acétylcystéine-6,7-dihydro-7-hydroxy-1-hydroxyméthyl-5H-pyrrolizine ; de la S-(2-(N(7)-guanyl)éthyl)-N-acétylcystéine ; de l'acétylcystéine(asp araginyl-alanyl-asparaginyl-proline)S ; de la S-trichlorovinyl-N-acétylcystéine ; de la S-(2-méthylbenzyl)-N-acétylcystéine ; de la S-1,2-dichlorovinyl-N-acétylcystéine ; du 2-(acétylcystéine)-N-isopropylacétanilide ; de la S-nitroso-N-acétylcystéine ; et de la N-acétylcystéine déacétylase.

**3.** Composition à utiliser selon la revendication 2, dans laquelle ladite NAC est du 2-acétamido-3-sulfanylpropanoïque.

**4.** Composition à utiliser selon la revendication 1, dans laquelle ledit polyphénol est sélectionné à partir du groupe constitué par : du 3-galactoside de cyanidine ; du 3-glucoside de cyanidine ; du s-O-glucoside de cyanidine ; du 3-arabinoside de cyanidine ; du 3-O-alpha-L-arabinopyranoside de cyanidine ; du 3-galactoside de péonidine ; du 3-glucoside de péonidine; du 3-arabinoside de péonidine ; du 3-xyloside de myricétine ; du 3-arabinoside de myricétine ; du 3-galactoside de quercétine ; du 3-xyloside de quercétine ; du 3-arabinopyranoside de quercétine ; du 3-arabinofuranoside de quercétine ; du 3-rhamnoside de quercétine ; de la myricétine ; du 3-xyloside de méthoxyquercétine ; du 3-coumaroyle galactoside de quercétine ; de la quercétine ; du 3-benzoyle galactoside de quercétine ; du 3-arabinoside de pétunidine ; du 3-O-alpha-L-arabinopyranoside de pétunidine ; de l'idaéine ; du 3-rhamnoside de delphinidine ; du 3-O-bêta-D-galactopyranoside de péonidine ; de l'oxycoccicyanine ; du 3-arabinoside de malvidine et n'importe quelle combinaison de ceux-ci.

**5.** Composition à utiliser selon la revendication 1, sous la forme d'un gel.

6. Composition à utiliser selon la revendication 1, sous la forme d'un bain de bouche.

7. Composition à utiliser selon l'une quelconque des revendications précédentes, dans laquelle ladite maladie parodontique est sélectionnée à partir de n'importe laquelle d'une gingivite, d'une mucosite péri-implantaire, d'une parodontite, d'une parodontite chronique, d'une parodontite agressive, d'une parodontite en tant que manifestation d'une maladie systémique, d'une parodontite ulcéro-nécrotique, des abcès du parodonte, des lésions parodontiques-endodontiques combinées et d'une péri-implantite.

8. Composition à utiliser selon l'une quelconque des revendications précédentes, sous une forme appropriée pour une administration sous-gingivale.

9. Composition à utiliser selon la revendication 6, dans laquelle ledit vecteur est un vecteur liquide.

10. Composition à utiliser selon l'une quelconque des revendications précédentes comprenant entre 5 % à 60 % de NAC et entre 0,04 % à 30 % d'un extrait de canneberge en poids de la composition.

11. Composition à utiliser selon la revendication 10, dans laquelle ladite combinaison comprend 20 % de NAC et entre 2,5 % et 5 % d'extrait de canneberge en poids de la composition finale.

12. Composition comprenant une combinaison synergique de N-acétylcystéine (NAC) et d'au moins un polyphénol de canneberge et un vecteur pharmaceutiquement acceptable pour une application orale topique de ladite combinaison, dans laquelle ladite combinaison comprend entre 5 % et 60 % de NAC et entre 0,04 % et 30 % d'extrait de canneberge en poids de la composition finale.

13. Composition selon la revendication 12, dans laquelle ladite combinaison comprend entre 5 % et 20 % de NAC et entre 0,04 % et 5 % d'extrait de canneberge en poids de la composition finale.

14. Composition selon la revendication 12, dans laquelle ladite combinaison comprend entre 15 % et 30 % de NAC et entre 0,8 % à 10 % d'extrait de canneberge en poids de la composition finale.

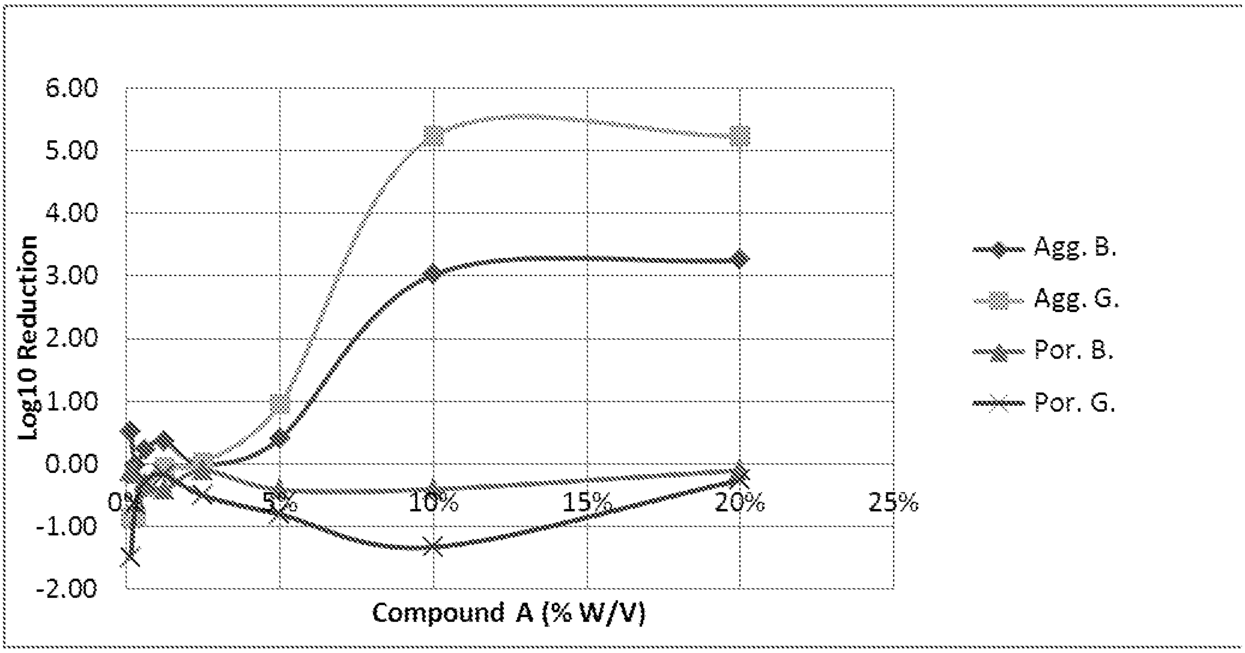
6.00
5.00
4.00
3.00
2.00
1.00
0.00
-1.00
-2.00
Log10 Reduction
0%
5%
10%
15%
20%
25%
Compound A (% W/V)
Agg. B.
Agg. G.
Por. B.
Por. G.

Fig. 1

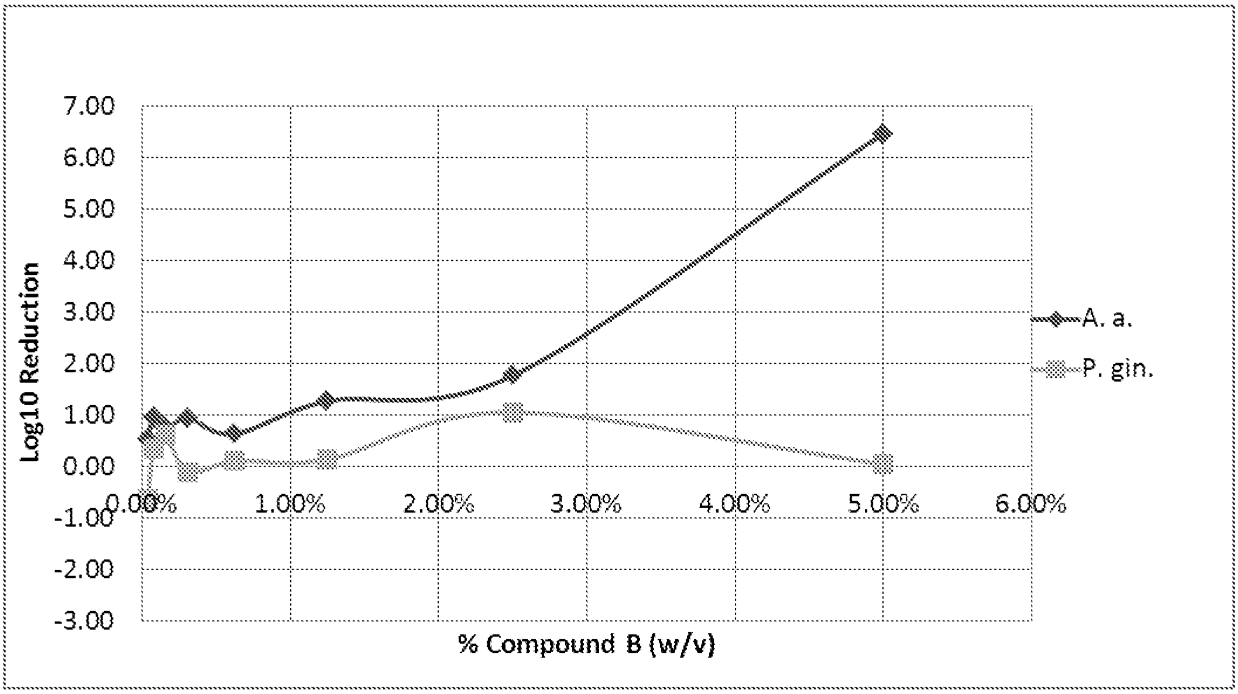
7.00
6.00
5.00
4.00
3.00
2.00
1.00
0.00
-1.00
-2.00
-3.00
Log10 Reduction
0.00%
1.00%
2.00%
3.00%
4.00%
5.00%
6.00%
% Compound B (w/v)
A. a.
P. gin.

Fig. 2

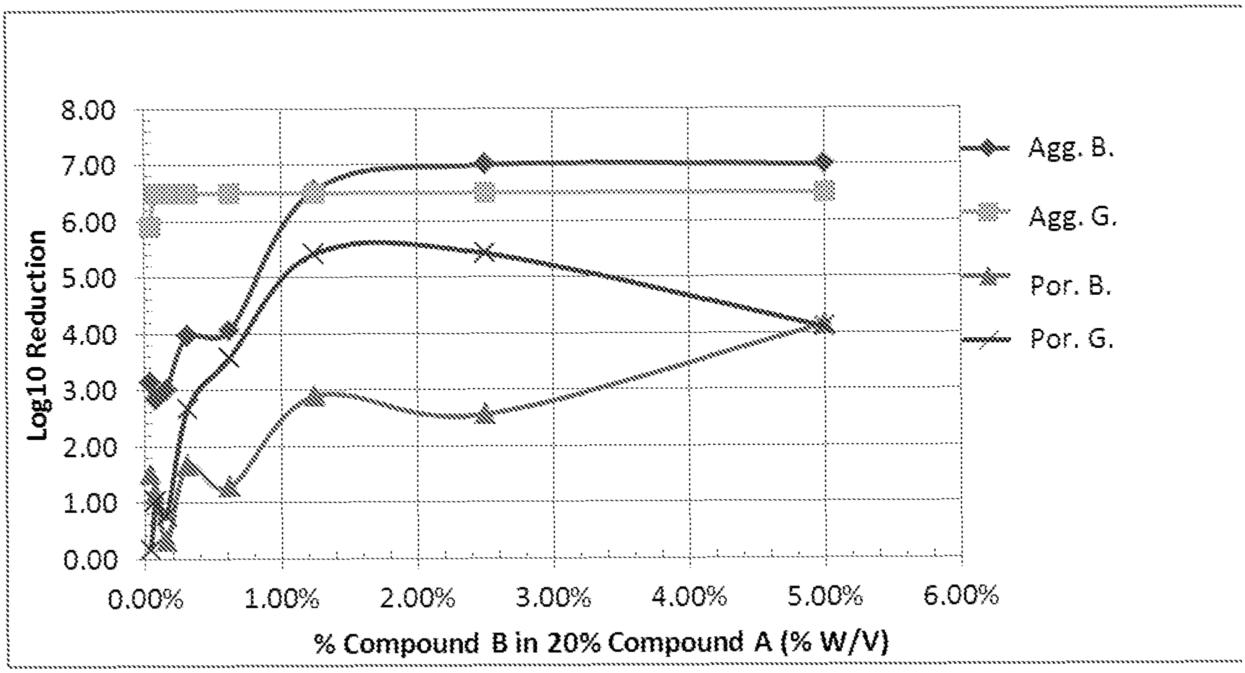
8.00
7.00
6.00
5.00
4.00
3.00
2.00
1.00
0.00
Log10 Reduction
0.00%
1.00%
2.00%
3.00%
4.00%
5.00%
6.00%
% Compound B in 20% Compound A (% W/V)
Agg. B.
Agg. G.
Por. B.
Por. G.

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BONIFAIT, L ; GRENIER D.** *J Can Dent Assoc,* 2010, vol. 76, a130